# EUROPEAN PATENT SPECIFICATION

(11) **EP 1 390 368 B1**
(45) Date of publication and mention of the grant of the patent: **26.10.2005**
(21) Application number: 01947360.2
(22) Date of filing: 08.06.2001
(51) Int. Cl.: C07D 473/18, A61K 31/52, A61P 35/00

(54) **DISUBSTITUTED 7,9-GUANINIUM HALIDES AS TELOMERASE INHIBITORS**
DISUBSTITUIERTE 7,9-GUANINIUM HALOGENIDE ALS TELOMERASEINHIBITOREN
HALOGENURES DE 7,9-GUANINIUM DISUBSTITUE UTILISES COMME DES INHIBITEURS DE TELOMERASE

(30) Priority: 04.05.2001 GB 0111072
(43) Date of publication of application: 25.02.2004
(73) Proprietor: Pharmacia Italia S.p.A., 20152 Milano (IT)
(72) Inventor: BARGIOTTI, Alberto, I-20146 Milano (IT); ERMOLI, Antonella, I-20090 Buccinasco (Milano) (IT); MENICHINCHERI, Maria, 20100 Milano (IT); VANOTTI, Ermes, I-20148 Milano (IT); BONOMINI, Luisella, I-20031 Cesano Maderno (Milano) (IT); FRETTA, Antonella, I-20094 Corsico (Milano) (IT)
(74) Representative: Modiano, Micaela Nadia
(86) International application number: PCT/EP2001/006625
(87) International publication number: WO 2002/090358

(56) References cited:
- WO-A-00/06573
- WO-A-01/02394
- WO-A-99/65875

## Description

The present invention relates to compounds that inhibit telomerase activity, to a process for their preparation, to their use as medicaments and to pharmaceutical compositions comprising them.

### BACKGROUND OF THE INVENTION

Cancer is one of the major causes of disease and the second leading cause of death in the western world. Most cancer patients still die due to metastatic disease. Despite the great increase in the knowledge and understanding of the regulatory mechanisms involved in the onset of malignancy, currently available treatments (including surgery, radiation and a variety of cytoreductive and hormone-based drugs, used alone or in combination, are still highly non specific and toxic to the patient, causing severe side effects including nausea and vomiting, hair loss, diarrhea, fatigue and ulcerations. These problems evidence the need for new and more effective anti-cancer therapies.

Recently an understanding of the mechanisms by which normal cells reach the state of replicative senescence, i.e. the loss of proliferative capacity that cells normally undergo in the cellular aging process has begun to emerge and in this respect telomerase appears to have a central role.

Telomerase is a ribonucleoprotein enzyme responsible in most eukaryotes for the complete replication and maintenance of chromosome ends, or telomeres, which are composed of repeated DNA sequences (in particular human telomeres are formed by 5'-TTAGGG repeats). Telomerase binds to telomeric DNA using as a template a sequence contained within the RNA component of the enzyme necessary for the addition of the short sequence repeats to the chromosome 3' end (see Blackburn 1992, Annu. Rev. Biochem., 61, 113-129). In most human somatic cells telomerase activity cannot be detected and telomeres shorten with successive cell division: in fact actively dividing normal cells have the potential to lose 50-200 base pairs after each round of cell division, resulting in shortening of telomeres. Recently it has been hypothesized that the cumulative loss of telomeric DNA over repeated cell divisions may act as a trigger for cellular senescence and aging, and that regulation of telomerase may have important biological implications (see Harley 1991, Mutation *Research,* 256, 271-282). In fact in the absence of telomerase, telomeres shortening will eventually lead to cellular senescence by various mechanisms. This phenomenon, thought to be responsible for cellular aging, is termed the "mitotic clock" (see Holt et al. *Nat.* Biotechnol., 1996, 15, 1734-1741). WO 99/65875 refers to methods and compositions for inhibiting telomerase activity using certain derivatives of isatin. WO 01/02394 refers to the use of certain indole derivatives to inhibit telomerase activity.

Telomerase activity is restored in immortalised cell lines and in more than 85% of human tumors, thus maintaining telomeres length stable (see Shay, J. W. and Bacchetti, S. Eur. J. Cancer, 1997, 33, 787-791). Thus in cancer cells having telomerase activity and where the malignant phenotype is due to the loss of cell cycle or growth controls or other genetic damage, telomeric DNA is not lost during cell division and telomers are maintained, thereby allowing the cancer cells to become immortal, leading to a terminal prognosis for the patient.

Telomerase inhibition can lead to telomere shortening in tumors and subsequent senescent phenotype (see Feng et al. *Science,* 1995, 269, 1236-1241). Moreover it has been recently shown (Hahn et al. *Nature Med.,* 1999, 5, 1164-1170) that inhibition of telomerase activity by expressing in tumor cells a catalytically-inactive form of human TERT (TElomerase Reverse Transcriptase, the catalytic subunit of the enzyme) can cause telomere shortening and arrest of cell growth and apoptosis. In addition peptide-nucleic acids and 2'-O-MeRNA oligomers complementary to the template region of the RNA component of the enzyme have been reported to cause inhibition of telomerase activity, telomere shortening and cell death in certain tumor cell lines (see Herbert et al. *PNAS,* 1999, 96, 14276-14281; Shammas et al. *Oncogene,* 1999, 18, 6191-6200). These data support inhibition of telomerase activity as an innovative, selective and useful method for the development of new anticancer agents.

Thus compounds that inhibit telomerase activity can be used to treat cancer, as cancer cells express telomerase activity, while normal human somatic cells usually do not express telomerase activity at biologically relevant levels (i.e., at levels sufficient to maintain telomere length over many cell divisions). Also telomere length in tumors is reduced compared with non-transformed cells giving the possibility of a therapeutic window (see Nakamura et al. *Cancer Letters* 158, 2000, 179-184). Therefore a need exists to find molecules that inhibit the activity of telomerase and interfere with the growth of many types of cancer.

The present invention fulfills such a need by providing a highly general method of treating many - if not most - malignancies, as demonstrated by the highly varied human tumor cell lines and tumors having telomerase activity. Since the compounds of the present invention can be effective in providing treatments that discriminate between malignant and normal cells to a high degree, avoiding many of the deleterious side-effects present with most current chemotherapeutic regimes which rely on agents that kill dividing cells indiscriminately, they are also expected to exhibit greater safety and lack of toxic effects in comparison with traditional chemotherapeutic anticancer agents.

### SUMMARY OF THE INVENTION

The present invention relates to novel purinic derivatives active as telomerase inhibitors, to their use as therapeutic agents, in particular as antitumoral agents, to a process for their preparation and to pharmaceutical compositions comprising them. These and other aspects of the invention are described in greater detail below.

### DETAILED DESCRIPTION OF THE INVENTION

Object of the present invention are 7,9 disubstituted guaninium halides of formula (I) 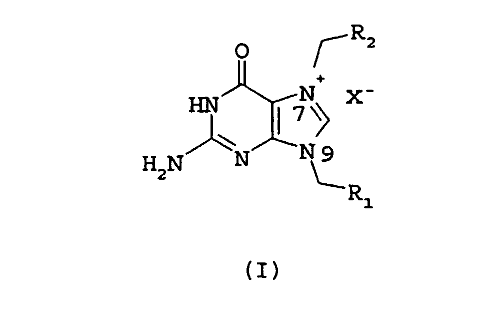 wherein
X is halogen; and
R1 and R2 represent each independently: unsubstituted phenyl; phenyl substituted by from 1 to 3 substituents chosen from halogen, unsubstituted phenyl and phenyl substituted by halo C₁-C₆ alkyl; or naphthyl.

The compounds described herein contain a ketonic carbonyl group as part of a heterocyclic ring system. Such carbonyl group may exist in part or principally in the "keto" form and in part or principally as "enol" form of the ketone group present. Compounds of the present invention are meant to include both "keto" and "enol" tautomeric forms. The compounds described herein contain one or more "imine" or "enamine" groups or combinations thereof. Such groups may exist in part or principally in the "imine" form and in part or principally as one or more "enamine" forms of each group present. Compounds of the present invention having said "imine" or "enamine" groups are meant to include both "imine" and "enamine" tautomeric forms. It is therefore understood that the present invention includes in its scope all the possible tautomeric forms of the compounds of formula (I) represented by the following tautomeric formulae (Ia)-(Id) : 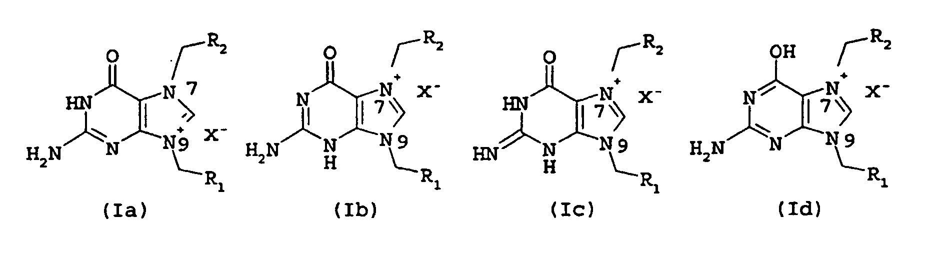 wherein R₁ and R₂ are as defined above. Accordingly, the chemical compounds provided by the present invention are named throughout the description of the invention according to the chemical nomenclature provided for the compounds of formula (I), (Ia)-(Id) on the basis of the structural evidence validated by people skilled in the art. The compounds of formula (I), (Ia)-(Id) are herein defined as the "compounds of the present invention", the "compounds of the invention" and/or the "active principles of the pharmaceutical compositions of the invention".

By the term "halogen" or "halo" as used herein, is meant chlorine, bromine, iodine or fluorine, preferably it is chlorine or fluorine.

C1-C6 alkyl is, preferably, C1-C4 alkyl, in particular methyl or ethyl.

Preferred compounds of the invention are compounds of formula (I) as defined above wherein X is halogen; and R₁ and R₂ represent, each independently, phenyl substituted by halogen or unsubstituted phenyl; or naphthyl.

Examples of preferred compounds of the invention are:
2-amino-7,9-bis(2-naphthylmethyl)-6-oxo-6,9-dihydro-1H-purin-7-ium bromide (compound 1);
2-amino-7,9-bis [(1,1'-biphenyl)-4-ylmethyl]-6-oxo-6,9-dihydro-1H-purin-7-ium bromide (compound 2);
2-amino-7,9-bis{[4'- (chloromethyl) [1,1'-biphenyl]-4-yl]methyl}-6-oxo-6,9-dihydro-1H-purin-7-ium bromide (compound 3);
2-amino-7,9-bis(3,4-dichlorobenzyl)-6-oxo-6,9-dihydro-1H-purin-7-ium bromide (compound 4);
2-amino-7,9-dibenzyl-6-oxo-6,9-dihydro-1H-purin-7-ium bromide (compound 5);
2-amino-9-[(1,1'-biphenyl)-4-ylmethyl]-7-(2-naphthylmethyl)-6-oxo-6,9-dihydro-1H-purin-7-ium bromide (compound 6); and
2-amino-7-([1,1'-biphenyl]-4-ylmethyl)-9-(2-naphthylmethyl)-6-oxo-6,9-dihydro-1H-purin-7-ium bromide (compound 7).

It is another object of the present invention a method for inhibiting telomerase enzyme, which comprises contacting said enzyme with an effective amount of a compound having the above formula(I).

It is a further object of the present invention a method for treating a telomerase-modulated disease, which comprises administering to a mammal a therapeutic effective amount of a compound having the above formula (I).

It is a still further object of the present invention a method for treating a cancer disease related to a deranged cancer cell growth mediated by telomerase enzyme activity, which comprises administering to a mammal a therapeutic effective amount of a compound having the above formula (I).

It is still another object of the present invention a method for treating a cancer, which comprises administering to a mammal a therapeutic effective amount of a compound having the above formula (I).

According to still another aspect of the invention, a method is provided which involves the use of a compound having the above formula (I) in the preparation of a medicament. In particular embodiments, the medicament is for treating a proliferative disorder (e.g. a cancer). The present invention therefore also provides a compound having the above formula (I) for use in the preparation of a medicament having anticancer activity.

A still further object of the present invention is to provide a pharmaceutical composition comprising a pharmaceutically acceptable carrier and/or diluent and, as an active principle, a compound of formula (I) as defined above.

The present invention also provides the use of a compound of formula (I) as defined above, in the preparation of a medicament for use as antitumor agent.

According to the present invention, there is provided a process for the preparation of 7,9 disubstituted guaninium halides of formula (I) as defined above.

A compound of formula (I) can be prepared by a process comprising:
the reaction of the compound of formula (II) 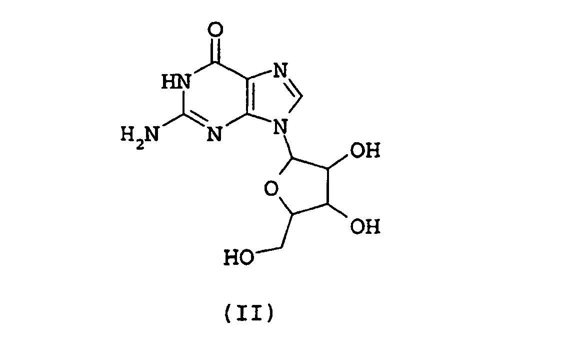 with a compound of formula (III) 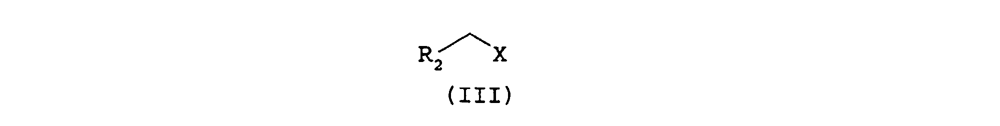 wherein
   X is a suitable leaving group and R₂ is as defined above, to obtain a compound of formula (IV) 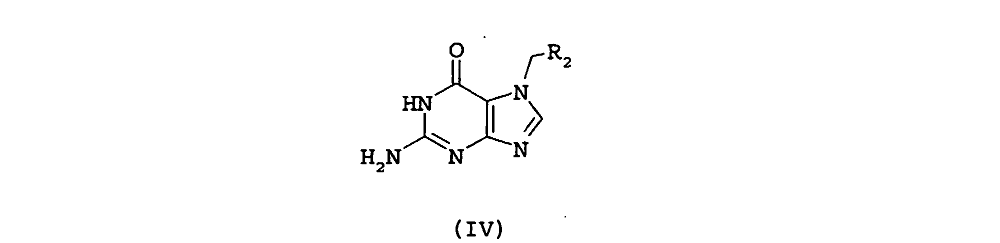 wherein R₂ is as defined above; and
   the reaction of a compound of formula (IV) with a compound of formula (V) 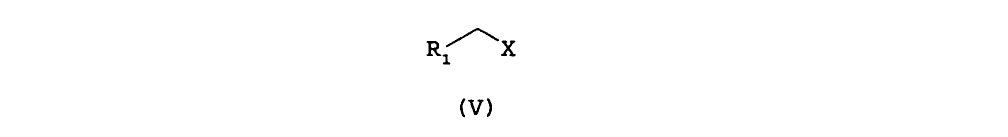 wherein
   X is a suitable leaving group and R₁=R₂ or, more preferably, wherein R₁ is different from R₂ as defined above, so obtaining a compound of formula (I).

A suitable leaving group is, e.g., a halogen, preferably Cl, Br or I; tosylate; mesylate; trifluoroacetate or triflate.

When the reaction between a compound of formula (II) as defined above and a compound of formula (III) as defined above is performed in dimethylsulfoxide (DMSO) or DMF, at a temperature varying between about 20°C and about 60°C, for a time of from about 1 hour to about 6 hours, as described, for example, in *J. Org. Chem.* 1986, 4180 or *Synth. Comm*. 1990, 2459, a monosubstituted derivative of formula (IV) as defined above can be obtained and used in the preparation of both symmetrical (where R₁=R₂) and unsymmetrical compounds of formula (I) (where R₁ is different from R₂) as described above. The reaction between a compound of formula (IV), and a compound of formula (V), may be carried out, for example, in a suitable organic solvent such as, e.g., N,N-dimethylacetamide, dimethylformamide (DMF), tetrahydrofuran, dioxane, dimethoxyethane or toluene, at a temperature varying between about 60°C and about 120°C, for a time of about 1 hour to about 15 hours, following, for example, literature methods as reported in *J.Med.Chem.* 1980, 357.

Examples of compounds prepared accordingly to this procedure are compounds 1, 6, 7.

A compound of formula (IV) can be also represented by the following tautomeric formulae (IVa), (IVb), (IVc) and (IVd): 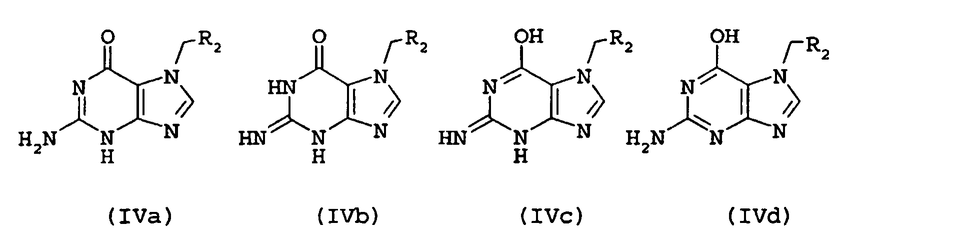 wherein R₂ is as defined above.

A compound of formula (I) wherein R₁=R₂ are as defined above can alternatively be prepared by a process comprising the reaction of a compound of formula (II) as defined above, with a compound of formula (III) as defined above in a suitable organic solvent such as, e.g., N,N-dimethylacetamide, dimethylformamide (DMF), tetrahydrofuran, dioxane, dimethoxyethane or toluene, at a temperature varying between about 60°C and about 120°C, for a time of about 1 hour to about 15 hours, following, for example, literature methods as reported in *J.Med.Chem.* 1980, 357.

Examples of compounds prepared accordingly to this procedure are compounds 1- 5.

Compounds of formula (II) and formula (III) are known compounds or can be prepared by known methods. For example, the compound of formula (II), which is the natural product guanosine and a compound of formula (III) can be prepared following methods well known in the art. For example, a compound of formula (III) can be prepared by halogenation reaction, with many methods known to people skilled in the art, starting from the corresponding alcohols that are commercially available or alternatively can be prepared, for example, from the corresponding commercial esters, by standard methods.

The compounds of the invention can be administered in a variety of dosage forms, e.g. orally, in the form of tablets, capsules, lozenges, liquid solutions or suspensions; rectally, in the form of suppositories; parenterally, e.g. intramuscularly, intravenously, intradermally or subcuteneously; or topically.

The dosage depends upon, for example, the compound of the invention employed, the age, weight, condition of the patient and administration route; specific dosage regimens may be fit to any particular subject on the basis of the individual need and the professional judgement of the person administering or supervising the administration of the aforesaid compounds.

For humans, therapy with the disclosed compounds includes doses of a pharmaceutical formulation comprising one or more of the compounds of the invention that are from about 0.001 to about 100 mg/kg. Preferably, the dosage is about 0.1 to 10 mg/kg. The dosages will vary in accordance with, for example, the condition of the patient and the type of disease being treated. A dosage can be administered once or can be divided into a number of smaller doses to be administered at varying intervals of time.

Pharmaceutical compositions containing, as an active ingredient, a compound of formula (I) or a pharmaceutically acceptable salt thereof in association with a pharmaceutically acceptable carrier and/or diluent, are also within the scope of the present invention.

These pharmaceutical compositions contain an amount of active ingredient, which is therapeutically effective to display antileukemic and/or antitumor activity. There may also be included as a part of the pharmaceutical compositions according to the invention, pharmaceutically acceptable binding agents and/or adjuvant materials. The active ingredients may also be mixed with other active principles, which do not impair the desired action and/or supplement the desired action.

The pharmaceutical compositions containing the compounds of the invention are usually prepared following conventional methods and may be administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents, e.g. starches, arabic gums, gelatin, methylcellulose, microcrystalline cellulose, carboxymethylcellulose or polyvinyl pyrrolidone; diaggregating agents, e.g. a starch, alginic acid, alginates or sodium starch glycolate; effervescing mixtures; dyestuffs; sweetening agents, e.g. sucrose or saccharin; flavouring agents, e.g. peppermint, methylsalicylate or orange flavouring; wetting agents, such as lecithin, polysorbates, laurylsulphates; and, in general, non-toxic and pharmacologically inactive substances used in pharmaceutical formulations. When the dosage unit form is a capsule, it may contain, in addition to material of the above type, a liquid carrier such as, e.g., a fatty oil.

Said pharmaceutical preparations may be manufactured in known manner, for example, by means of mixing, granulating, tabletting, sugar-coating or film-coating processes. The liquid dispersions for oral administration may be, e.g. syrups, emulsions and suspensions. The syrups may contain as carrier, for example, saccharose or saccharose with glycerine and/or mannitol and/or sorbitol; in particular, a syrup to be administered to diabetic patients can contain as carriers only products not metabolizable to glucose, or metabolizable in very small amount to glucose, for example sorbitol.

The suspensions and the emulsions may contain as carrier, for example, a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride.

The solutions for intravenous injections or infusions may contain as carrier, for example, sterile water, or preferably they may be in the form of sterile, aqueous, isotonic saline solution.

The solutions or suspensions for parenteral therapeutic administration may also contain antibacterial agents, such as benzyl alcohol or methyl parabens; antioxidants, such as ascorbic acid or sodium bisulphite; chelating agents, such as ethylenediaminetetraacetic acid; buffers, such as acetates, citrates or phosphates and agents for the adjustment of tonicity, such as sodium chloride or dextrose.

The parenteral preparation can be enclosed in ampoules, disposable syringes or multiple dose vials made of glass or plastic.

The suppositories may contain together with the active compound a pharmaceutically acceptable carrier, e.g., coca-butter, polyethylene glycol, a polyoxyethylene sorbitan fatty acid ester surfactant or lecithin.

Compositions for topical application, such as, e.g., creams, lotions or pastes, may be, e.g., prepared by admixing the active ingredient with a conventional oleaginous or emulsifying excipient.

### Biological activity

The telomerase activity of the compounds of the invention has been evaluated using a Flash Plate-based assay. The method proved to be sensitive, accurate and able to reproducibly identify compounds that inhibit telomerase activity in a dose-dependent manner. Other methods for determining the inhibitory concentration of a compound of the invention against telomerase can be employed as will be apparent to a person skilled in the art based on the disclosure herein.

Briefly, the assay mixture is costituted by:
- telomerase enzyme diluted in a buffer, the composition of which has been selected to maintain the enzyme activity stable along the duration of the assay.
- dNTPs, deoxynucleotides 5'-triphosphate.
- biotynilated oligo as primer.
- increasing concentrations of test compounds/positive control.

After two hours of incubation at 37° degrees the telomeric repeats added are evaluated by hybridization in solution with a 3'-labeled short oligonucleotide probe.

The extent of hybridization is then quantitated by transferring the reaction mixture in a streptavidin-coated flash plate, where the binding between biotin and streptavidin occurs.

The telomerase activity is proportional to the radioactivity measured and the inhibitory activity of the compounds is evaluated as IC₅₀ using the Sigma Plot fit program.
With the above-described method, IC₅₀ values of the compounds of the present invention were determined.
The results relative to a representative selection of compounds of the invention are shown in the following Table 1.

**Table 1**

| Compound | IC₅₀ (µM) |
|---|---|
| 1 | 10 |
| 2 | 3 |
| 4 | 10 |
| 7 | 4 |

The data reported in Table 1 clearly show the activity of the compounds according to the invention as telomerase inhibitors.

A human or animal body may thus be treated by a method, which comprises the administration thereto of a pharmaceutically effective amount of a compound of formula (I) or a salt thereof. The condition of the human or animal can thereby be improved.

The compounds of the invention can be administered either as single agents or, alternatively, in combination with one or more anti-cancer agent including, for example, topoisomerase inhibitors, antimetabolites, alkylating agents, antibiotics, antimicrotubule agents, hormonal agents, immunological agents, interferon-type agents, cyclooxygenase inhibitors (e.g. COX-2 inhibitors), metallomatrixprotease inhibitors, kinase inhibitors, tyrosine kinase inhibitors, anti-growth factor receptor agents, anti-HER agents, anti-EGFR agents, farnesyl transferase inhibitors, ras-raf signal transduction pathway inhibitors, cell cycle inhibitors, tubulin binding agents and anti-angiogenesis agents.

Combinations of drugs are administered in an attempt to obtain a synergistic effect on most cancers, e.g., carcinomas, melanomas, sarcomas, lymphomas and leukemias and/or to reduce or eliminate emergence of drug-resistant cells and/or to reduce side effects to each drug.

It is therefore a still further aspect of the present invention a combined anti-cancer therapy which comprises administering a compound according to the invention with at least one other anti-cancer agent. The combined use of active substances provides improved therapeutic effect than employing the single agents alone.

Compounds of formula (I) may be combined with at least one other anti-cancer agent in a fixed pharmaceutical formulation or may be administered with at least one other anti-cancer agent in any desired order.

It is therefore a further object of the invention a product or kit comprising a compound of formula (I) of the invention and one or more anti-cancer agents for simultaneous, separate or sequential use in anticancer therapy.

Anti-cancer agents suitable for combination with the compounds of the present invention include, but are not limited to:
- topoisomerase I inhibitors comprising, for example, epipodophyllotoxins such as, e.g. etoposide and teniposide; camptothecin and camptothecin derivatives including, e.g., irinotecan, SN-38, topotecan, 9-amino-camptothecin, 10,11-Methylenedioxy camptothecin and 9-nitro-camptothecin (rubitecan);
- alkylating agents including nitrogen mustards such as, e.g., mechlorethamine, chlorambucil, melphalan, uracil mustard and estramustine; alkylsulfonates such as, e.g., busulfan improsulfan and piposulfan; oxazaphosphorines such as e.g., ifosfamide, cyclophosphamide, perfosfamide, and trophosphamide; platinum derivatives such as, e.g., oxaliplatin, carboplatin and cisplatin; nitrosoureas such as, e.g., carmustine, lomustine and streptozocin;
- antimitotic agents including taxanes such as , e.g., paclitaxel and docetaxel; vinca alkaloids such as, e.g., vincristine, vinblastine, vinorelbine and vindesine; and novel microtubule agents such as, e.g., epothilone analogs, discodermolide analogs and eleutherobin analogs;
- antimetabolites including purines such as , e.g., 6-mercaptopurine, thioguanine, azathioprine, allopurinol, cladribine, fludarabine, pentostatin, and 2-chloro adenosine; fluoropyrimidines such as, e.g., 5-FU, fluorodeoxyuridine, ftorafur, 5'-deoxyfluorouridine, UFT, S-1 and capecitabine; and pyrimidine nucleosides such as, e.g., deoxycytidine, cytosine arabinoside, 5-azacytosine, gemcitabine, and 5-azacytosine-arabinoside; antimetabolites (for example antifolates like methotrexate, fluoropyrimidines like 5-fluorouracil, purine and adenosine analogues, cytosine arabinoside;
- hormones, hormonal analogues and hormonal antagonists including antiestrogens (for example tamoxifen, toremifen, raloxifene, droloxifene and iodoxyfene), progestogens (for example megestrol and acetate), aromatase inhibitors (for example anastrozole, letrazole, borazole and exemestane), antiprogestogens, antiandrogens (for example flutamide, nilutamide, bicalutamide and cyproterone acetate), LHRH agonists and antagonists (for example gosereline acetate and luprolide) and inhibitors of testosterone 5a-dihydroreductase (for example finasteride;
- antitumor antibiotics including anthracyclines and anthracenediones such as, e.g., doxorubicin, daunorubicin, epirubicin, idarubicin and mitoxantrone;
- farnesyltransferase inhibitors including, for example, SCH 44342, RPR 113228, BZA 5B and PD 161956;
- anti-invasion agents (for example metalloproteinase inhibitors such as, e.g., marimastat and inhibitors of urokinase plasminogen activator receptor functions);
- inhibitors of growth factor (for example, EGF, FGF, platelet derived growth factor and hepatocyte growth factor) functions including growth factor antibodies, growth factor receptor antibodies, tyrosine kinase inhibitors and serine/threonine kinase inhibitors;
- antiangiogenic agents such as, for example, linomide, inhibitors of integrin avβ3 function, angiostatin, razoxin, SU 5416, SU 6668, AGM 1470 (TNP-470), a synthetic analogue of fumagillin a naturally secreted product of the fungus Aspergillus fumigates fresenius, platelet factor 4 (endostatin), thalidomide, marimastat (BB-2516) and batimastat (BB-94);
- cyclooxygenase (COX) inhibitors, preferably COX-2 inhibitors such as, for example, celecoxib, parecoxib, rofecoxib, valecoxib and JTE 5222; and
- cell cycle inhibitors such as, e.g., flavopyridols.

In a further aspect of this invention, a method is provided for treating a cancer comprising administering to a patient in need of such treatment a therapeutically effective amount of a compound of formula (I) as defined above and a therapeutically effective amount of at least another anti-cancer agent. In a preferred aspect of the present invention, the combination of a compound of formula (I) as defined above with at least one antiangiogenesis agent is contemplated.

The terms "malignant neoplasm", "cancer", "tumor" and "solid tumor cancer" are used interchangeably herein to refer to the condition well known to those skilled in the art as the life-threatening disease commonly referred to simply as "cancer". The term "cancer" as used herein, is meant a disease characterized by excessive, uncontrolled growth of abnormal cells, which invades and destroys other tissues and includes all human cancers such as carcinomas, sarcomas, leukemias and lymphomas. For example, the term "cancer" comprises prostate, breast, lung, colorectal, bladder, uterine, skin, kidney, pancreatic, ovarian, liver and stomach cancer.

By the term "chemotherapeutic agent" as used herein, is meant a chemical substance or drug used to treat a disease; the term is most often applied to such substances or drugs which are used primarily for the treatment of cancer.

By the term "treating" as used herein, is meant reversing, alleviating, ameliorating, limiting, inhibiting the progress of, or preventing the disorder or condition to which such term applies, or one or more symptoms of such disorder or condition. The term "treatment" as used herein, refers to the act of treating as "treating" is defined immediately above.

By the term "method" as used herein, is meant manners, means, techniques and procedures for accomplishing a given task including, but not limited to, those manners, means, techniques and procedures either known to, or readily developed from known manners, means, techniques and procedures by, practitioners of the chemical, pharmacological, biological, biochemical and medical arts.

By the term "administered" or "administering" s used herein, is meant standard delivery methods, e.g, parenteral administration, including continuous infusion and intravenous, intramuscular and subcutaneous injections, and oral administration.

The term "modulated" as used herein includes governed, controlled provoked, modulated and induced.

By the term "mammal" as used herein, is meant any of a class of warm-blooded higher vertebrates, that nourish their young with milk secreted by mammary glands, have the skin usually more or less covered with hair, and include humans.

By the term "pharmaceutically acceptable carrier" as used herein, is meant a carrier or diluent that does not cause significant irritation to an organism and does not abrogate the biological activity and properties of the administered compound.

By the term "excipient" as used herein, is meant an inert substance added to a pharmaceutical composition to further facilitate administration of a compound.

By the term "disease" as used herein, is meant a kind or instance of impairment of a living being that interferes with normal bodily function.
The following examples are provided for exemplification purposes only and are not intended to limit the scope of the invention described in broad terms above.

### Example 1: compound 1

A mixture of guanosine hydrate (2.83 g; 10 mmols) and 2-naphthylmethyl bromide (4.41 g; 20 mmols) in N,N-dimethylacetamide (100mL) is stirred at 90°C for 3 hours. After solvent evaporation under reduced pressure the crude reaction product is purified by flash chromatography (eluant: dichloromethane /methanol 10:1) to yield 2-amino-7,9-bis(2-naphthylmethyl)-6-oxo-6,9-dihydro-1H-purin-7-ium bromide as a white solid. Yield: 40%.
- ¹H-NMR (400Mhz, DMSOd₆), ppm:: 5.5 (2H, s), 5.8 (2H, s), 6.95 (2H, s), 7.4-8.0 (m, 14H), 9.35 (1H, s).

By analogous procedure the following compounds were prepared:
2-Amino-7,9-bis[(1,1'-biphenyl)-4-ylmethyl]-6-oxo-6,9-dihydro-1H-purin-7-ium bromide (compound 2).
   - ¹H-NMR (400Mhz, DMSOd₆), ppm:: 5.4 (2H, s), 5.65 (2H, s), 7.2 (2H, bs), 7.2-7.8 (18H, m), 9, 5 (1H, s), 11.7 (1H, bs).
2-Amino-7,9-bis{[4'-(chloromethyl)[1,1'-biphenyl]-4-yl]methyl}-6-oxo-6,9-dihydro-1H-purin-7-ium bromide (compound 3).
   - ¹H-NMR (400Mhz, DMSOd₆), ppm:: 4.65 (4H, s), 5.25 (2H, s), 5.65 (2H, s), 7.1-7.7 (17H, m), 9.3 (1H, s), 11.8 (1H, bs).
2-Amino-7,9-bis(3,4-dichlorobenzyl)-6-oxo-6,9-dihydro-1H-purin-7-ium bromide (compound 4).
   - ¹H-NMR (400Mhz, DMSOd₆), ppm:: 5.3 (2H, s), 5.6 (2H, s), 7.1 (2H, bs), 7.2-7.8 (6H, m), 9.3 (1H, s).
2-Amino-7,9-dibenzyl-6-oxo-6,9-dihydro-1H-purin-7-ium bromide (compound 5).
   - ¹H-NMR (400Mhz, DMSOd₆), ppm:: 5.3 (2H, s), 5.6 (2H, s), 6.95 (2H, bs), 7.2-7.5 (10H, m), 9.3 (1H, s).

### Example 2: compound 1

To a solution of guanosine hydrate (570mg; 2mmol) in anhydrous DMSO (3mL) under argon 2-naphthylmethyl bromide (1.075g; 4.8mmol) is added and the reaction mixture stirred at room temperature for 4h. Concentrated aq. HCl (1.5mL) is added, the mixture is stirred for 0.5h then poured into methanol (20mL). The solution is concentrated and added of dichloromethane/methanol 2:1 (5mL). The white precipitate is filtered and washed with dichloromethane. In this way 7-(2-naphthylmethyl) guanine hydrochloride (90% yield) is obtained and used for the second step. 7-(2-Naphthylmethyl) guanine hydrochloride (200mg; 0.62mmol) and 2-naphthylmethyl bromide (144mg; 0.65mmol) are suspended in N,N-dimethylacetamide (15mL) and the mixture is stirred at 90°C for 3h. The solvent is evaporated under reduced pressure and the crude reaction product is purified by flash chromatography (eluant dichloromethane/methanol 10:1) to yield 2-amino-7,9-bis(2-naphthylmethyl)-6-oxo-6,9-dihydro-1H-purin-7-ium bromide as a white solid. Yield: 35%.

By analogous procedure the following compound was prepared:
2-Amino-7-([1,1'-biphenyl]-4-ylmethyl)-9-(2-naphthylmethyl)-6-oxo-6,9-dihydro-1H-purin-7-ium bromide (compound 7).
   - ¹H-NMR (400Mhz, DMSOd₆), ppm:: 5.5 (2H, s), 5.65 (2H, s), 7.2 (2H, bs), 7.3-8.0 (16H, m), 9.5 (1H, s).

### Example 3: compound 6

7-(2-naphthylmethyl) guanine hydrochloride (200mg; 0.62mmol) and (1,1'-biphenyl)-4-ylmethyl chloride (132mg; 0.65mmol) are suspended in N,N-dimethylacetamide (15mL) and the mixture is stirred at 120°C for 8h. The solvent is evaporated under reduced pressure and the crude reaction product is purified by flash chromatography (eluant dichloromethane/methanol 10:1) to yield 2-amino-9-[(1,1'-biphenyl)-4-ylmethyl]-7-(2-naphthylmethyl)-6-oxo-6,9-dihydro-1H-purin-7-ium bromide as a white solid (31% yield).
¹H-NMR (400Mhz, DMSOd₆), ppm: 5.3 (2H, s), 5.7 (2H, s), 7.2 (2H, s), 7.3-7.8 (16H, m), 9.35 (1H, s).

### Example 4: Intramuscular injection of 50mg/ml

A pharmaceutical injectable composition can be manufactured by dissolving 50 g of 2-amino-7,9-bis(2-naphthylmethyl)-6-oxo-6,9-dihydro-1H-purin-7-ium bromide (compound 1) in sterile propylene glycol (1000 ml) and sealed in 1-5 ml ampoules.

## Claims

1. 7,9 disubstituted guaninium halides of formula (I) 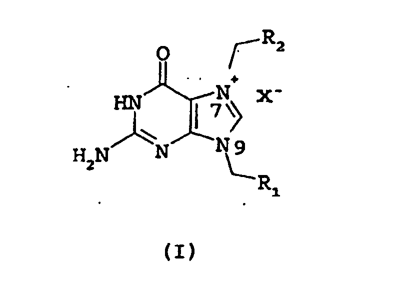 wherein
X is halogen; and
R1 and R2 represent each independently: unsubstituted phenyl; phenyl substituted by from 1 to 3 substituents chosen from halogen, unsubstituted phenyl and phenyl substituted by halo C₁-C₆ alkyl; or naphthyl.

2. A compounds of formula (I) as claimed in claim 1 wherein X is halogen; and
R₁ and R₂ represent each independently: phenyl substituted by halogen or unsubstituted phenyl; or naphthyl.

3. A compound selected from the group consisting of:
2-amino-7,9-bis(2-naphthylmethyl)-6-oxo-6,9-dihydro-1H-purin-7-ium bromide (compound 1);
2-amino-7,9-bis[(1,1'-biphenyl)-4-ylmethyl)-6-oxo-6,9-dihydro-1H-purin-7-ium bromide (compound 2);
2-amino-7,9-bis{[4'-(chloromethyl)[1,1'-biphenyl]-4-yl)methyl}-6-oxo-6,9-dihydro-1H-purin-7-ium bromide (compound 3);
2-amino-7,9-bis(3,4-dichlorobenzyl)-6-oxo-6,9-dihydro-1H-purin-7-ium bromide (compound 4);
2-amino-7,9-dibenzyl-6-oxo-6,9-dihydro-1H-purin-7-ium bromide (compound 5);
2-amino-9-[(1,1'-biphenyl)-4-ylmethyl]-7-(2-naphthylmethyl)-6-oxo-6,9-dihydro-1H-purin-7-ium bromide (compound 6); and
2-amino-7-([1,1'-biphenyl]-4-ylmethyl)-9- (2-naphthylmethyl)-6-oxo-6,9-dihydro-1H-purin-7-ium bromide (compound 7).

4. Use of a compound having the formula (I) as defined in claim 1, for manufacturing a medicament for inhibiting telomerase enzyme.

5. Use of a compound having the formula (I) as defined in claim 1, for manufacturing a medicament for the treatment of a telomerase-modulated desease.

6. Use of a compound having the formula (I) as defined in claim 1, for manufacturing a medicament for the treatment of a cancer desease related to a deranged cancer cell growth mediated by telomerase enzyme activity.

7. Use of a compound having the formula (I) as defined in claim 1, for manufacturing a medicament for the treatment of a cancer.

8. A pharmaceutical composition comprising a pharmaceutically acceptable carrier and/or diluent and, as an active principle, a compound of formula (I) as defined in claim 1.

9. A process for preparing a compound of formula (I) as defined in claim 1 comprising:
the reaction of the compound of formula (II) 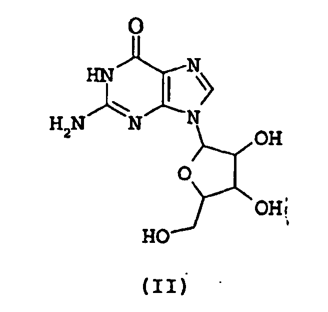 with a compound of formula (III) 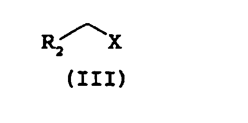
wherein
X is a suitable leaving group and R₂ is as defined above, to obtain a compound of formula (IV) 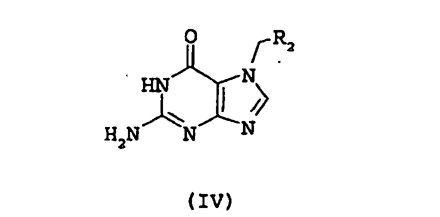 wherein R₂ is as defined above; and
the reaction of a compound of formula (IV) with a compound of formula (V) 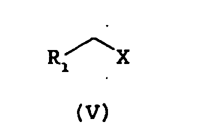 wherein
X is a suitable leaving group and R₁=R₂ or, more preferably, wherein R₁ is different from R₂ as defined above, so obtaining a compound of formula (I).

10. Use of a compound of formula (I) as defined in claim 1 and at least another anticancer agent, for manufacturing a medicament for the improved treatment of a cancer.

11. A kit comprising a compound of formula (I) as defined in claim 1, and one or more anti-cancer agents for simultaneous, separate or sequential use in anticancer therapy.

## Patentansprüche

1. 7,9 distubsituierte Guaniniumhalogenide der Formel (I) 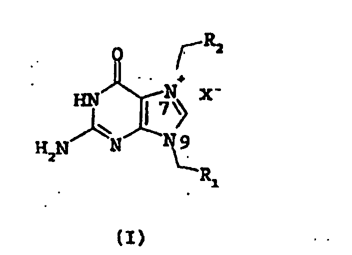 worin
X Halogen ist; und
R1 und R2 stellen jeweils unabhängig folgendes dar: unsbustituiertes Phenyl; Phenyl substituiert mit von 1 bis 3 Substituenten, gewählt aus Halogen, unsubstituiertem Phenyl und Phenyl substituiert mit Halo C₁-C₆ Alkyl; oder Naphthyl.

2. Eine Verbindung mit der Formel (I), wie in Anspruch 1 beansprucht, worin X Halogen ist; und R₁ und R₂ stellen jeweils unabhängig folgendes dar: Phenyl substituiert mit Halogen oder unsubstituiertem Phenyl; oder Naphthyl.

3. Eine Verbindung gewählt aus der Gruppe bestehend aus:
2-Amino-7,9-bis(2-naphthylmethyl)-6-oxo-6,9-dihydro-1H-purin-7-ium Bromid (Verbindung 1);
2-Amino-7,9-bis[(1,1'-biphenyl)-4-ylmethyl]-6-oxo-6,9-dihydro-1H-purin-7-ium Bromid (Verbindung 2);
2-Amino-7,9-bis{[4'-(chlormethyl)[1,1'-biphenyl]-4-yl]methyl}-6-oxo-6,9-dihydro-1H-purin-7-ium Bromid (Verbindung 3);
2-Amino-7,9-bis(3,4-dichlorbenzyl)-6-oxo-6,9-dihydro-1H-purin-7-ium Bromid (Verbindung 4);
2-Amino-7,9-dibenzyl-6-oxo-6,9-dihydro-1H-purin-7-ium Bromid (Verbindung 5);
-2-Amino-9-[(1,1'-biphenyl)-4-ylmethyl]-7-(2-naphthylmethyl)-6-oxo-6,9-dihydro-1H-purin-7-ium Bromid (Verbindung 6); und
2-Amino-7-([1,1'-biphenyl]-4ylmethyl)-9-(2-naphthylmethyl)-6-oxo-6,9-dihydro-1H-purin-7-ium Bromid (Verbindung 7).

4. Verwendung einer Verbindung, welche die Formel (I) hat, wie in Anspruch 1 definiert, für die Herstellung eines Medikaments zur Hemmung von Telomeraseenzym.

5. Verwendung einer Verbindung, welche die Formel (I) hat, wie in Anspruch 1 definiert, für die Herstellung eines Medikaments für die Behandlung einer Telomerase-modulierten Erkrankung.

6. Verwendung einer Verbindung, welche die Formel (I) hat, wie in Anspruch 1 definiert, für die Herstellung eines Medikaments für die Behandlung einer Krebserkrankung, die in Zusammenhang mit einem gestörten Krebszellwachstum steht, vermittelt durch Telomeraseenzym-Aktivität.

7. Verwendung einer Verbindung, welche die Formel (I) hat, wie in Anspruch 1 definiert, für die Herstellung eines Medikaments für die Behandlung von Krebs.

8. Eine pharmazeutische Zusammensetzung, die einen pharmazeutisch verträglichen Träger und/oder Verdünnungsmittel und, als einen aktiven Stoff, eine Verbindung von Formel (I) wie in Anspruch 1 definiert, umfasst.

9. Ein Verfahren zur Herstellung einer Verbindung von Formel (I), wie in Anspruch 1 definiert, das folgendes umfaßt:
die Reaktion der Verbindung von Formel (II) 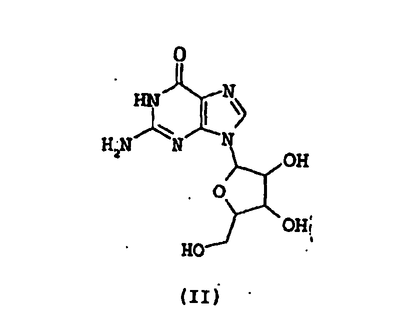 mit einer Verbindung von Formel (III) 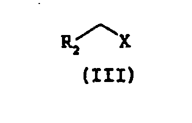 worin
X eine geeignete Austrittsgruppe ist, und R₂ ist wie oben definiert, um eine Verbindung von Formel (IV) zu erhalten 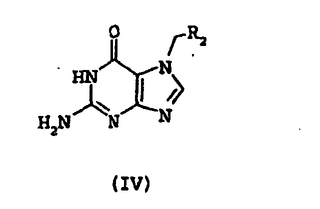 worin R₂ wie oben definiert ist; und
die Reaktion einer Verbindung von Formel (IV) mit einer Verbindung von Formel (V) 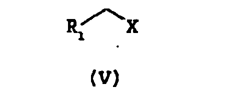 worin
X eine geeignete Austrittsgruppe ist, und R₁=R₂ oder, bevorzugter, worin R₁ anders ist als R₂ wie oben definiert, um eine Verbindung der Formel (I) zu erhalten.

10. Verwendung einer Verbindung von Formel (I), wie in Anspruch 1 definiert, und mindestens eines anderen Antikrebsmittels, für die Herstellung eines Medikaments für die verbesserte Behandlung von Krebs.

11. Ein Kit, umfassend eine Verbindung von Formel (I) wie in Anspruch 1 definiert, und ein oder mehrere Antikrebsmittel für die gleichzeitige, separate oder aufeinanderfolgende Verwendung in der Antikrebstherapie.

## Revendications

1. Halogénures de guaninium 7,9-disubstitués de formule (I) 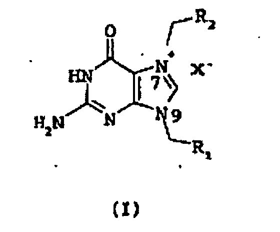 dans laquelle :
X représente un atome d'halogène ; et
R₁ et R₂ représentent chacun indépendamment : un groupe phényle non substitué ; un groupe phényle substitué avec 1 à 3 substituants choisis entre des substituants halogéno, phényle non substitué et phényle substitué avec un substituant halogénalkyle en C₁ à C₆ ; ou un groupe naphtyle.

2. Composés de formule (I) suivant la revendication 1, dans lesquels X représente un atome d'halogène ; et
R¹ et R² représentent chacun indépendamment : un groupe phényle substitué avec un substituant halogéno, ou un groupe phényle non substitué ; ou un groupe naphtyle.

3. Composé choisi dans le groupe consistant en :
le bromure de 2-amino-7,9-bis(2-naphtyméthyl)-6-oxo-6,9-dihydro-1H-purine-7-ium (composé 1) ;
le bromure de 2-amino-7,9-bis[(1,1'-biphényl)-4-ylméthyl]-6-oxo-6,9-dihydro-1H-purine-7-ium (composé 2) ;
le bromure de 2-amino-7,9-bis{[4'-(chlorométhyl)[1,1'-biphényl]-4-yl]méthyl}-6-oxo-6,9-dihydro-1H-purine-7-ium (composé 3) ;
le bromure de 2-amino-7,9-bis(3,4-dichlorobenzyl)-6-oxo-6,9-dihydro-1H-purine-7-ium (composé 4) ;
le bromure de 2-amino-7,9-dibenzyl-6-oxo-6,9-dihydro-1H-purine-7-ium (composé 5) ;
le bromure de 2-amino-9-[(1,1'-biphényl)-4-ylméthyl]-7-(2-naphtylméthyl)-6-oxo-6,9-dihydro-1H-purine-7-ium (composé 6) ;
le bromure de 2-amino-7-([1,1'-biphényl]-4-ylméthyl)-9-(2-naphtylméthyl)-6-oxo-6,9-dihydro-1H-purine-7-ium (composé 7).

4. Utilisation d'un composé répondant à la formule (I) telle que définie dans la revendication 1, pour la production d'un médicament destiné à inhiber l'enzyme télomérase.

5. Utilisation d'un composé répondant à la formule (I) telle que définie dans la revendication 1, pour la production d'un médicament destiné au traitement d'une maladie à modulation par la télomérase.

6. Utilisation d'un composé de formule (I) telle que définie dans la revendication 1, pour la production d'un médicament destiné au traitement d'une maladie cancéreuse en rapport avec une croissance cellulaire cancéreuse perturbée à médiation par l'activité de l'enzyme télomérase.

7. Utilisation d'un composé de formule (I) telle que définie dans la revendication 1, pour la production d'un médicament destiné au traitement d'un cancer.

8. Composition pharmaceutique comprenant un support et/ou diluant pharmaceutiquement acceptable et, comme principe actif, un composé de formule (I) telle que définie dans la revendication 1.

9. Procédé pour la préparation d'un composé de formule (I) telle que définie dans la revendication 1, comprenant :
la réaction du composé de formule (II) 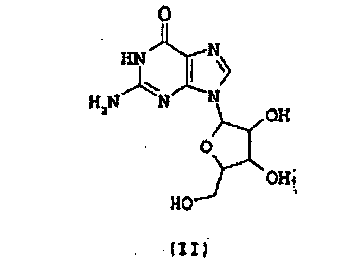 avec un composé de formule (III) 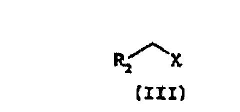 dans laquelle
X représente un groupe partant convenable et R₂ répond à la définition précitée, pour obtenir un composé de formule (IV) 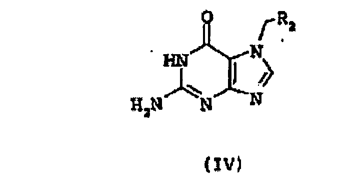 dans laquelle R₂ répond à la définition précitée ; et
la réaction d'un composé de formule (IV) avec un composé de formule (V) 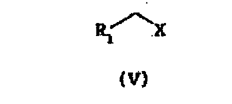 dans laquelle
X représente un groupe partant convenable et R₁=R₂ ou, plus avantageusement, R₁ est différent de R₂ de la manière définie ci-dessus, de manière à obtenir un composé de formule (I).

10. Utilisation d'un composé de formule (I) telle que définie dans la revendication 1 et d'au moins un autre agent anticancéreux, pour la production d'un médicament destiné au traitement amélioré d'un cancer.

11. Kit comprenant un composé de formule (I) telle que définie dans la revendication 1 et un ou plusieurs agents anticancéreux pour une utilisation simultanée, séparée ou séquentielle en thérapie anticancéreuse.
